(19) Europäisches Patentamt
European Patent Office
Office européen des brevets

(11) Veröffentlichungsnummer : **0 053 603**
**B1**

(12) # EUROPÄISCHE PATENTSCHRIFT

(45) Veröffentlichungstag der Patentschrift : **03.04.85**

(51) Int. Cl.[4] : **C 07 D333/32,** A 61 K 31/38

(21) Anmeldenummer : **81890190.2**

(22) Anmeldetag : **17.11.81**

(54) **1-(3-(2-Hydroxy-3-alkylaminopropoxy)-2-thienyl)-3-phenyl-1-propanone und ihre Säureadditionssalze sowie Verfahren zu deren Herstellung.**

(30) Priorität : **28.11.80 AT 5818/80**

(43) Veröffentlichungstag der Anmeldung : **09.06.82 Patentblatt 82/23**

(45) Bekanntmachung des Hinweises auf die Patenterteilung : **03.04.85 Patentblatt 85/14**

(84) Benannte Vertragsstaaten : **BE CH DE FR GB IT LI LU NL SE**

(56) Entgegenhaltungen : **DE-A- 2 720 613**
**Die Akte enthält technische Angaben, die nach dem Eingang der Anmeldung eingereicht wurden und die nicht in dieser Patentschrift enthalten sind.**

(73) Patentinhaber : **Laevosan-Gesellschaft m.b.H. & Co KG**
**Estermannstrasse 17**
**A-4020 Linz (AT)**

(72) Erfinder : **Binder, Dieter, Univ. Prof., Dr.**
**Sieveringerstrasse 207**
**A-1190 Wien (AT)**

(74) Vertreter : **Pawloy, Heinrich, Dr. et al**
**Riemergasse 14**
**A-1010 Wien (AT)**

**EP 0 053 603 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung bezieht sich auf neue Verbindungen, nämlich Derivate des 1-[3-(2-Hydroxy-3-alkylaminopropoxy)-2-thienyl]-3-phenyl-1-propanons, die wertvolle therapeutische Eigenschaften besitzen, und auf ein Verfahren zu deren Herstellung.

Insbesondere bezieht sich die Erfindung auf Verbindungen der allgemeinen Formel I

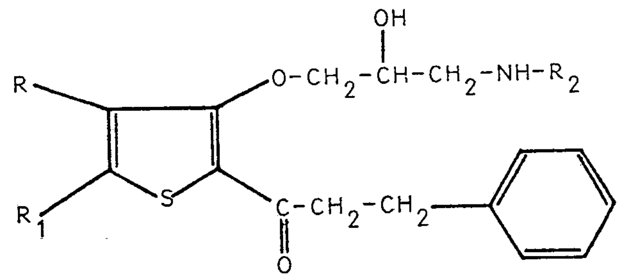

(I)

worin R und $R_1$ Wasserstoff oder Methyl und $R_2$ n-Propyl, n-butyl, Isobutyl oder tert.-Butyl bedeuten, und ihre therapeutisch verwendbaren Säureadditionssalze.

Die neuen Verbindungen der allgemeinen Formel I besitzen an verschiedenen Tiermodellen schon in niedrigen Dosen hervorragende antiarrhythmische Aktivität und sind auch oral wirksam.

Eine bevorzugte Klasse sind Verbindungen der allgemeinen Formel I, worin $R_2$ n-Propyl bedeutet.

Aufgrund dieser pharmakologischen Eigenschaften können die neuen Verbindungen bzw. ihre therapeutisch verwendbaren Säureadditionssalze allein oder in Mischung mit anderen Wirksubstanzen in Form üblicher galenischer Zubereitungen (auch Retardformen) bei Herzrhythmusstörungen als Medikament angewendet werden.

Das erfindungsgemäße Verfahren zur Herstellung der Verbindungen der allgemeinen Formel I und ihrer therapeutisch verwendbaren Säureadditionssalze ist dadurch gekennzeichnet, daß man eine Verbindung der allgemeinen Formel II

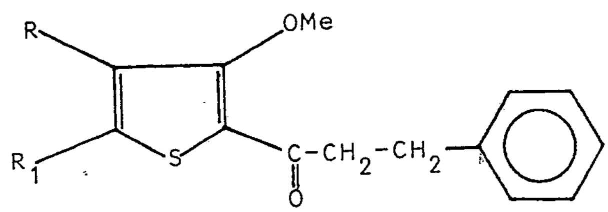

(II)

worin R und $R_1$ die obige Bedeutung besitzen und Me ein Alkalimetall-Kation bedeutet, mit Epichlorhydrin der Formel III

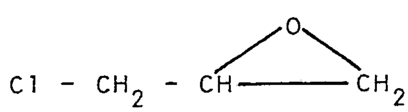

(III)

umsetzt und die dabei erhaltene Verbindung der allgemeinen Formel IV

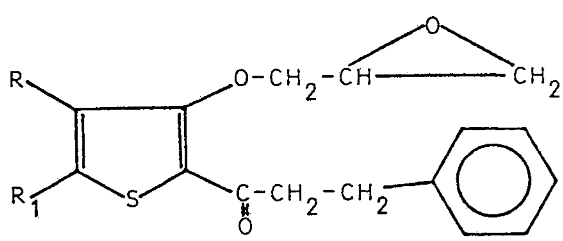

(IV)

worin R und $R_1$ die obige Bedeutung besitzen, mit einem Alkylamin der allgemeinen Formel V

$$R_2\text{—}NH_2 \qquad (V)$$

worin $R_2$ die obige Bedeutung besitzt, umsetzt und die so erhaltene freie Base der allgemeinen Formel I gegebenenfalls in ein therapeutisch verwendbares Säureadditionssalz überführt.

Vorzugsweise wird die Umsetzung der Verbindung II mit dem Epichlorhydrin in einem reaktionsinerten Lösungsmittel oder insbesondere in überschüssigem Epichlorhydrin bei einer Temperatur zwischen 110 und 140 °C durchgeführt.

Vorzugsweise wird die Umsetzung der Verbindung IV mit dem Alkylamin V in einem reaktionsinerten Lösungsmittel oder insbesondere in überschüssigem Amin V bei einer Temperatur zwischen 50 und 100 °C durchgeführt.

Die Säureadditionssalze der Endverbindungen können in an sich bekannter Weise, z. B. mit Alkalien oder Ionenaustauschern, in die freien Basen übergeführt werden, von denen sich durch Umsetzung mit anorganischen oder organischen Säuren, die zur Bildung von therapeutisch verwendbaren Salzen geeignet sind, andere Salze gewinnen lassen.

Infolge der engen Beziehungen zwischen freier Base und deren Säureadditionssalzen sind im vorausgegangenen und nachfolgenden sinn- und zweckmäßig gegebenenfalls auch unter den freien Basen die entsprechenden Säureadditionssalze zu verstehen.

Die Ausgangsmaterialien der Formel III und V sind literaturbekannt.

Verbindungen der allgemeinen Formel II können aus den freien Verbindungen (Me = H) am besten mittels Alkalihydroxiden, -hydriden oder -alkoholaten hergestellt werden.

Die Verbindungen der allgemeinen Formel II können, ausgehend von den literaturbekannten Verbindungen der allgemeinen Formel VI, nach folgendem Reaktionsschema hergestellt werden :

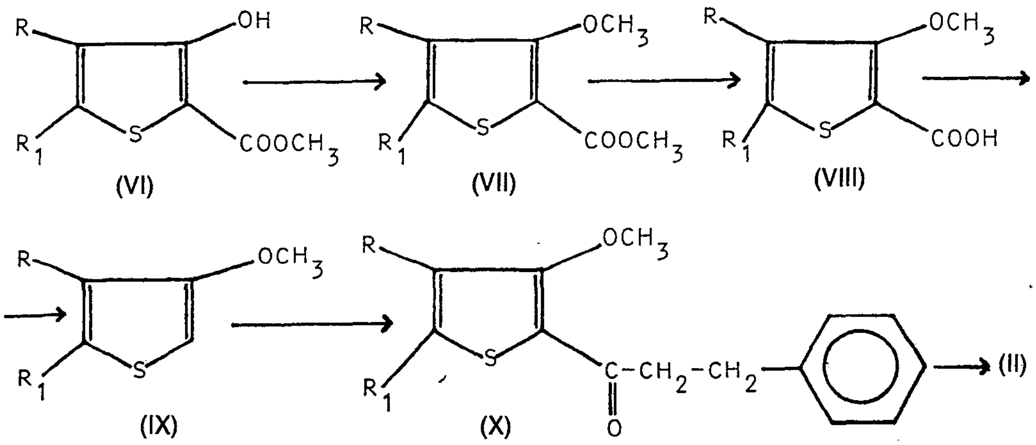

(VI) (VII) (VIII) (IX) (X) (II)

Das folgende Beispiel soll die Erfindung erläutern.

Beispiel

1-[3-(2,3-Epoxypropoxy)-2-thienyl]-3-phenyl-1-propanon (Formel IV : R und $R_1$ = H)

1,74 g (0,076 Mol) Na werden in 50 ml abs. Methanol gelöst, 17,55 g (0,076 Mol) 1-(3-Hydroxy-2-thienyl)-3-phenyl-1-propanon (Formel II : R und $R_1$ = H, Me = H) eingetragen und die Lösung zur Trockene (im Vakuum) eingedampft. Der kristalline Rückstand (Fomel II : R und $R_1$ = H ; Me = Na) wird mit 50 ml Epichlorhydrin (Formel III) versetzt und 4 h rückflußerhitzt. Die Reaktionsmischung wird über Hyflo abgesaugt, mit etwas Benzol nachgespült und das Filtrat im Vakuum eingedampft. Der kristalline Rückstand wird in 1 250 ml Cyclohexan unter Zugabe von Aktivkohle aufgekocht, über Hyflo abgesaugt und das Filtrat im Vakuum eingedampft. Der kristalline Rückstand wiegt 16,9 g (77,6 %). Das Produkt ist für den weiteren Einsatz rein genug. Fp. (Cyclohexan) : 59 bis 61 °C.

Analog erhält man :

1-[3-(2,3-Epoxypropoxy)-4-methyl-2-thienyl]-3-phenyl-1-propanon (Formel IV : R = $CH_3$, $R_1$ = H), Öl, nicht isoliert.

1-[3-(2,3-Epoxypropoxy)-5-methyl-2-thienyl]-3-phenyl-1-propanon (Formel IV : R = H, $R_1$ = $CH_3$), Öl, nicht isoliert.

1-[4,5-Dimethyl-3-(2,3-epoxypropoxy)-2-thienyl]-3-phenyl-1-propanon (Formel IV : R und $R_1$ = $CH_3$), Öl, nicht isoliert, $n_D^{20}$ : 1,553 5.

1-[3-(2-Hydroxy-3-n-propylaminopropoxy)-2-thienyl]-3-phenyl-1-propanon-chlorhydrat (Formel I : HCl.R und $R_1$ = H, $R_2$ = n-Propyl).

14,4 g (0,05 Mol) rohes 1-[3-(2,3-Epoxypropoxy)-2-thienyl]-3-phenyl-1-propanon (Formel IV : R und $R_1$ = H) werden in 40 ml n-Propylamin (Formel V : $R_2$ = Propyl) 4 h unter Rühren rückflußerhitzt. Die Reaktionsmischung wird im Vakuum eingedampft, der Rückstand zwischen 250 ml $CH_2Cl_2$ und 150 ml 1n HCl verteilt, die Phasen getrennt, die wässerige Phase noch zweimal mit je 50 ml $CH_2Cl_2$ extrahiert, die

vereinigten organischen Phasen getrocknet ($Na_2SO_4$) und im Vakuum eingedampft. Das kristallin anfallende Rohprodukt kann aus Aceton/Methanol 8 : 2 (ca. 120 ml) mit Aktivkohle umkristallisiert werden. Ausbeute : 12,2 g (64 %) farblose Kristalle, Fp. : 150 bis 152 °C.

Analog erhält man :

1-[3-(2-Hydroxy-3-n-butylaminopropoxy)-2-thienyl]-3-phenyl-1-propanon-chlorhydrat (Formel I : R und $R_1$ = H, $R_2$ = n-Butyl), Fp. (aus Aceton) : 113 bis 114 °C ;

1-[3-(2-Hydroxy-3-n-tert.-butylaminopropoxy)-2-thienyl]-3-phenyl-1-propanon-chlorhydrat (Formel I : R und $R_1$ = H ; $R_2$ = tert.-Butyl), Fp. (aus Aceton) : 143 bis 144 °C ;

1-[4-Methyl-3-(2-hydroxy-3-n-propylamino-propoxy)-2-thienyl]-3-phenyl-1-propanon-chlorhydrat (Formel I : R = $CH_3$, $R_1$ = H ; $R_2$ = n-Propyl) ; Fp. 107 bis 111 °C (Methanol/Äther).

1-[5-Methyl-3-(2-hydroxy-3-n-propylamino-propoxy)-2-thienyl]-3-phenyl-1-propanon-chlorhydrat (I : R = H, $R_1$ = $CH_3$ ; $R_2$ = n-Propyl) ; Fp. (aus Methanol) : 203 bis 205 °C ;

1-[4,5-DImethyl-3-(2-hydroxy-3-n-propylamino-propoxy)-2-thienyl]-3-phenyl-1-propanon-chlorhydrat (I : R und $R_1$ = $CH_3$, $R_2$ = n-Propyl), Fp. 126 bis 128 °C (Toluol/Äther).

Das Ausgangsprodukt kann folgendermaßen hergestellt werden :

3-Methoxy-thiophen-2-carbonsäure (Formel VIII : R und $R_1$ = H)

13,5 g (0,085 Mol) 3-Hydroxythiophen-2-carbonsäuremethylester (VI : R und $R_1$ = H) werden in eine Lösung von 36 g (0,34 Mol) $Na_2CO_3$ in 145 ml $H_2O$ eingetragen und unter Rühren kurz aufgekocht. Dann werden innerhalb von 15 min 21,53 g (0,17 Mol) Dimethylsulfat zugetropft, wobei die Temperatur des Reaktionsgemisches knapp unter dem Siedepunkt gehalten wird. Nach dem Ende der Zugabe wird noch 20 min unter Rückfluß erhitzt, die Reaktionsmischung abgekühlt und mit $CH_2Cl_2$ mehrmals extrahiert. Die vereinigten organischen Phasen werden dreimal mit 2n NaOH extrahiert, getrocknet ($Na_2SO_4$) und eingedampft. Der ölige Rückstand, bestehend aus 3-Methoxy-thiophen-2-carbonsäuremethylester (VII : R und $R_1$ = H) wiegt 7,1 g. Dieser wird in einer Lösung von 2,54 g (0,045 Mol) KOH in 70 ml Methanol gelöst und 30 min rückflußerhitzt. Dann wird das Methanol im Vakuum abdestilliert und der Rückstand zwischen Wasser und $CH_2Cl_2$ verteilt. Die wässerige Phase wird mit konz. HCl angesäuert und die ausfallenden farblosen Kristalle abgesaugt. Ausbeute : 5,7 g (54 %) farblose Kristalle ; Fp. : 180 bis 183 °C.

Analog erhält man :

3-Methoxy-4-methyl-thiophen-2-carbonsäure VIII : R = $CH_3$. $R_1$ = H, Fp. 119 bis 121 °C.

3-Methoxy-5-methyl-thiophen-2-carbonsäure VIII : R = H, $R_1$ = $CH_3$, Fp. 175 bis 177 °C.

4,5-Dimethyl-3-methoxy-thiophen-2-carbonsäure VIII : R und $R_1$ = $CH_3$, Fp. 133 bis 135 °C.

3-Methoxythiophen (IX : R und $R_1$ = H)

57 g 3-Methoxythiophen-2-carbonsäure (VIII : R und $R_1$ = H) werden mit 15 g Cu-Pulver verrieben und in einem Destillierkolben, der über eine Destillationsbrücke mit der Vorlage verbunden ist, überführt. An die Apparatur wird ein Vakuum von 65 mm herangelegt und der Destillationskolben auf 150 bis 180 °C erhitzt. Das abdestillierende 3-Methoxythiophen wird in der Vorlage aufgefangen, Ausbeute : 33 g (80 %).

Analog erhält man :

3-Methoxy-4-methyl-thiophen IX : R = $CH_3$, $R_1$ = H,
Kp. 83 °C/3 990 Pa ;

3-Methoxy-5-methyl-thiophen IX : R = H, $R_1$ = $CH_3$,
Kp. 63 bis 65 °C, 16 mbar,

4,5-Dimethoxy-3-methoxy-thiophen IX : R und $R_1$ = $CH_3$,
Kp. 78 bis 84 °C/1 330 Pa.

1-(3-Hydroxy-2-thienyl)-3-phenyl-2-propanon (II : R und $R_1$ = H)

In 400 ml abs. $CHCl_3$ werden 44,9 g (0,266 Mol) 3-Phenylpropionsäurechlorid gelöst und 69,37 g (0,266 Mol) $SnCl_4$ in 50 ml $CHCl_3$ zugetropft. Dann wird die Temperatur des Gemisches auf 15 °C erhöht und 30,4 g (0,266 Mol) 3-Methoxythiophen (IX : R und $R_1$ = H), gelöst in 250 ml $CHCl_3$, innerhalb von 30 min zugetropft und noch 20 min gerührt. Das Reaktionsgemisch wird auf halbkonz. HCl geschüttet, die organische Phase abgetrennt und mit $CH_2Cl_2$ noch zweimal extrahiert. Die vereinigten organischen Phasen werden dreimal mit $NaHCO_3$-Lösung extrahiert, getrocknet ($Na_2SO_4$) und eingedampft. Der ölige Rückstand (57 %), der überwiegend aus 1-(3-Methoxy-2-thienyl)-3-phenyl-1-propanon (X : R und $R_1$ = H) besteht, wird in 270 ml Nitrobenzol gelöst und in eine Lösung von 62 g wasserfreiem $AlCl_3$ in 270 ml Nitrobenzol eingetragen. Die Reaktionsmischung wird 1 1/2 h bei 70 bis 90 °C gerührt, abgekühlt, auf

Eis/HCl gegossen und über Nacht bei Raumtemperatur stehen gelassen. Dann werden die Phasen getrennt, die wässerige Phase noch zweimal mit $CH_2Cl_2$ extrahiert, die vereinigten organischen Phasen dreimal mit 2n NaOH extrahiert, die vereinigten NaOH-Phasen mit konz. HCl angesäuert und das ausfallende Produkt dreimal mit $CH_2Cl_2$ extrahiert. Nach Eindampfen der vereinigten $CH_2Cl_2$-Phasen wird der dunkle kristalline Rückstand in Benzol gelöst, über Kieselgel filtriert und gut nachgewaschen.

Nach Eindampfen des Filtrates werden 29,1 g (47 %) reines Produkt erhalten, Fp. (Methanol) : 50 °C.

Analog werden erhalten :

1-(3-Hydroxy-4-methyl-2-thienyl)-3-phenyl-1-propanon (II : R = $CH_3$, $R_1$ = H), Fp. 36 bis 38 °C (Methanol) ;

1-(3-Hydroxy-5-methyl-2-thienyl)-3-phenyl-1-propanon

(II : R = H, $R_1$ = $CH_3$), Öl, $n_D^{20}$ = 1,601 4 ;

1-(4,5-Dimethyl-3-hydroxy-2-thienyl)-3-phenyl-1-propanon

(II : R und $R_1$ = $CH_3$), Fp. (Toluol/Äther) : 43 bis 46 °C.

Bei der Untersuchung der antiarrhythmischen Wirksamkeit einer Verbindung wird der Schutz durch Abnahme des Auftretens ventrikulärer ektopischer Schläge oder ventrikulärer Fibrillation nach Verabreichung der zu testenden Verbindung gemessen. Je nach dem Protokoll bilden entweder die Tiere ihre eigene Kontrolle oder aber sie werden in zwei Gruppen, nämlich Kontrollgruppe und behandelte Gruppe, geteilt.

Die Verbindungen wurden zunächst auf Grund ihrer Wirksamkeit in verschiedenen experimentellen Arrhythmiemodellen, die als Auswähltechnologien angewendet werden, gewählt. So wurden der Chloroform-, der Aconitin- und der Calciumchloridtest zum Testen der erfindungsgemäßen Verbindungen verwendet, wie von J.W. Lawson, J. Pharmacol. exp. Ther. 160, 22-81 (1968) « Antiarrhythmic activity of some isoquinoline derivatives determined by a rapid screening procedure in the mouse » beschrieben.

Codenummern :

LG 80-6-00 = 1-[3-(2-Hydroxy-3-n-butylaminopropoxy)-2-thienyl]-3-phenyl-1-propanon-chlorhydrat

LG 80-6-01 = 1-[4-Methyl-3-(2-hydroxy-3-n-propylaminopropoxy)-2-thienyl]-3-phenyl-1-propanon-chlorhydrat

LG 80-6-02 = 1-[5-Methyl-3-(2-hydroxy-3-n-propylaminopropoxy)-2-thienyl]-3-phenyl-1-propanon-chlorhydrat

Prop. = Propafenon.

Chloroformtest :

5 min nach i.v. Injektion der zu untersuchenden Verbindung wurden die Mäuse in einen 300 ml-Becher eingebracht, der mit 20 ml Chloroform getränkte Watte enthielt. Die Tiere wurden sorgfältig beobachtet und aus dem Becher herausgenommen, sobald der Atem zum zweiten Male aussetzte. Dann wurde das Herz rasch visuell auf ventrikulären Rhythmus untersucht (siehe Lawson, loc. cit.). Die $ED_{50}$ ist die Dosis der Verbindungen, die 50 % der Mäuse gegen durch Chloroform induzierte ventrikuläre Fibrillation schützt. Die folgende Tabelle zeigt die $ED_{50}$ der neuen Verbindungen LG 80-6-00, LG 80-6-01, LG 80-6-02 und der Bezugsverbindung Propafenon.

Aconitintest :

Die Forschung dürfte Ratten bei der Untersuchung des schützenden Effektes einer Verbindung auf durch Aconitin induzierte Arrhythmie bevorzugen [siehe J.L. Junien et al., Arzneim. Forsch. 24, 1743-1747 (1974) ; L. Szekeres et al., Experimental cardiac arrhythmias and antiarrhythmic drugs, Publishing House of the Hungarian Academy of Sciences (Akadèmia Kiado), Budapest (1971) ; M.R. Malinow et al., Arch. Int. Pharmacodyn. 102, 55-64 (1955) ; C. Bianchi et al., Arzneim. Forsch. 18, 845-850 (1968) ; B.B. Vargaftig et al., J. Pharm. Pharmacol. 27, 697-699 (1975) ; und M. Fekete et al., Med. Exp. 10, 93-102 (1964)].

Die zu untersuchenden Verbindungen wurden der urethananästhesierten Ratte vor der Injektion von Aconitin verabreicht. Verschiedene antiarrhythmische Drogen können das Einsetzen von aconitininduzierten Arrhythmien verzögern und dieses Arrhythmie-Modell scheint zum Feststellen der antiarrhythmischen Wirksamkeit geeignet zu sein.

Die folgende Tabelle zeigt die Ergebnisse dieses Versuches hinsichtlich der $ED_{50}$ von LG 80-6-00, LG 80-6-01, LG 80-6-02 und Propafenon. Es ist ersichtlich, daß die beste Verbindung bei dieser Methode LG 80-6-01 ist.

Calciumchloridtest :

Cardiale Arrhythmien treten häufig dann auf, wenn die Spiegel von Serumionen abnormal sind, insbesondere wenn der Calciumspiegel viermal höher ist als die normale Konzentration. Dies ist der

Grund, warum intervenöse Verabreichung hoher Dosen von Calciumchlorid die Entwicklung von Dysrhythmien bei Tieren hervorrufen kann.

Bei der anästhesierten Ratte bewirkt die rasche intravenöse Injektion von Calciumchlorid letale ventrikuläre Fibrillation oder ventrikuläre Extrasystolen. Die Bewertung der Aktivität der vor der Calciumchloridinjektion verabreichten Testverbindung basiert auf dem Einsetzen von Arrhythmie [M.R. Malinow et al., Arch. int. Pharmacodyn. 102, 55-64 (1955), « The pharmacology of experimental ventricular arrhythmias in the rat I. Antihistaminic drugs »] oder der Rückkehr zum Sinusrhythmus nach gelegentlichen Rhythmusstörungen [M. Fekete et al., Med. Exp. 10, 93-102 (1964), « On the antiarrhythmic effect of some thymoleptics : amitriptyline, imipramine, trimepropimine and desmethylimipramine »].

Es hat sich gezeigt, daß die Verbindungen der Erfindung Schutzeffekte gegen durch Calciumchlorid induzierte Arrhythmien aufweisen. Die $ED_{50}$ der Verbindungen der Erfindung im Vergleich mit der Kontrollverbindung Propafenon sind in der folgenden Tabelle aufgeführt. Es ist ersichtlich, daß LG 80-6-01 den besten Schutz gegen durch Calciumchlorid induzierte Arrhythmien bietet.

Tabelle

| Testverbindung | $ED_{50}$ mg/kg Körpermasse | | |
|---|---|---|---|
| | Chloroform Maus | Aconitin Ratte | Calciumchlorid Ratte |
| LG 80-6-00 | 4,16 | 4,23 | 5,04 |
| LG 80-6-01 | 3,57 | 1,49 | 2,59 |
| LG 80-6-02 | 17,57 | 22,8 | 2,87 |
| Prop. | 3,45 | 2,10 | 4,49 |

**Ansprüche**

1. 1-[3-(2-Hydroxy-3-alkylaminopropoxy)-2-thienyl]-3-phenyl-1-propanone der allgemeinen Formel I

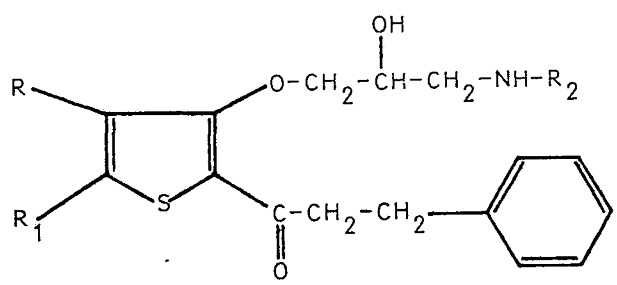

(I)

worin R und $R_1$ jeweils Wasserstoff oder Methyl und $R_2$ n-Propyl, n-Butyl, Isobutyl oder tert.-Butyl bedeuten, und deren therapeutisch verwendbare Säureadditionssalze.

2. Verfahren zur Herstellung der Verbindungen der allgemeinen Formel I und ihrer therapeutisch verwendbaren Säureadditionssalze, dadurch gekennzeichnet, daß man eine Verbindung der allgemeinen Formel II

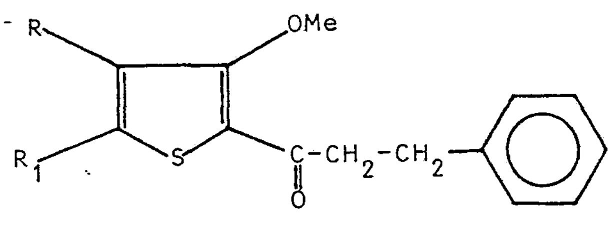

(II)

worin R und $R_1$ die obige Bedeutung besitzen und Me ein Alkalimetall-Kation bedeutet, mit Epichlorhydrin der Formel III

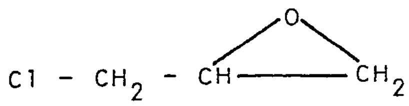

(III)

umsetzt und die dabei erhaltene Verbindung der allgemeinen Formel IV

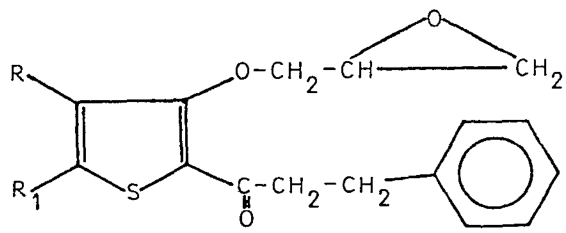

(IV)

worin R und $R_1$ die obige Bedeutung besitzen, mit einem Alkylamin der allgemeinen Formel V

$$R_2—NH_2 \qquad (V)$$

worin $R_2$ die obige Bedeutung besitzt, umsetzt und die so erhaltene Base der allgemeinen Formel I gegebenenfalls in ein therapeutisch verwendbares Säureadditionssalz überführt.

3. Verfahren nach Anspruch 2, dadurch gekennzeichnet, daß man die Umsetzung der Verbindung II mit dem Epichlorhydrin III in einem reaktionsinerten Lösungsmittel durchführt.

4. Verfahren nach Anspruch 3, dadurch gekennzeichnet, daß man die Umsetzung in überschüssigem Epichlorhydrin bei einer Temperatur zwischen 110 und 140 °C durchführt.

5. Verfahren nach Anspruch 2, dadurch gekennzeichnet, daß man die Umsetzung der Verbindung IV mit dem Alkylamin V in einem reaktionsinerten Lösungsmittel durchführt.

6. Verfahren nach Anspruch 5, dadurch gekennzeichnet, daß man die Umsetzung in überschüssigem Amin V bei einer Temperatur zwischen 50 und 100 °C durchführt.

7. Pharmazeutische Zubereitung, dadurch gekennzeichnet, daß sie einen therapeutisch wirksamen Anteil einer Verbindung der allgemeinen Formel I zusammen mit einem pharmazeutisch annehmbaren Träger oder Verdünnungsmittel enthält.

## Claims

1. 1-[3-(2-Hydroxy-3-alkylaminopropoxy)-2-thienyl]-3-phenyl-1-propanones of the formula I

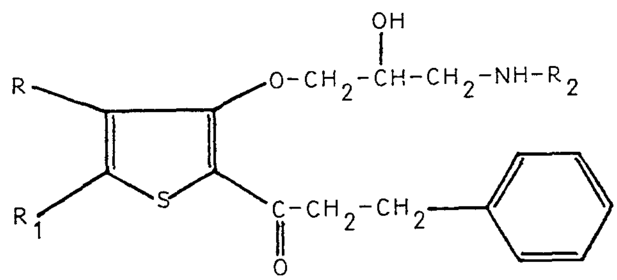

(I)

in which R and $R_1$ are independently hydrogen or methyl and $R_2$ is n-propyl, n-butyl, isobutyl or tert. butyl and the therapeutically useful acid addition salts thereof.

2. A process for preparing the compounds of the general formula I and the therapeutically useful acid addition salts, characterized in that a compound of the general formula II

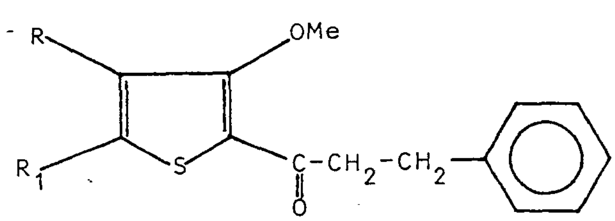

(II)

in which R and $R_1$ are as defined above and Me is an alkali metal cation is reacted with epichlorohydrin of the formula III

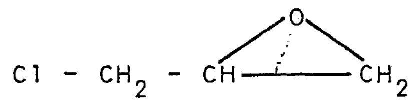 (III)

and that the compound of the general formula IV

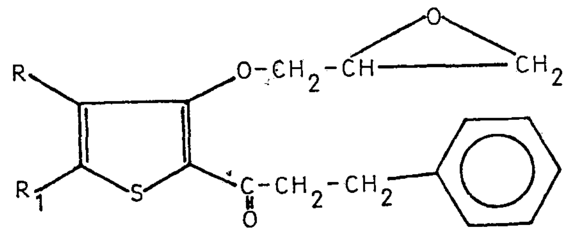 (IV)

thus obtained, in which R and $R_1$ are as defined above, is reacted with an alkyl amine of the general formula V

$$R_2\text{—}NH_2 \qquad (V)$$

in which $R_2$ is as defined above, and, if desired, an obtained base of the general formula I is converted to a therapeutically useful acid addition salt.

3. The process of claim 2, in which the reaction of compound II with the epichlorohydrin is carried out in an reaction-inert solvent.

4. The process of claim 3, in which the reaction is carried out in an excess of epichlorohydrin at a temperature between 110 and 140 °C.

5. The process of claim 2, in which the reaction of compound IV with the alkyl amine is carried out in an reaction-inert solvent.

6. The process of claim 5, in which the reaction is carried out in an excess of the amine V at a temperature between 50 and 100 °C.

7. Pharmaceutical composition comprising a therapeutically effective amount of a compound of general formula I together with a pharmaceutically acceptable carrier or diluent.

**Revendications**

1. 1-[3-(2-hydroxy-3-alkylaminopropoxy)-2-thiényl]-3-phényl-1-propanones de formule générale I

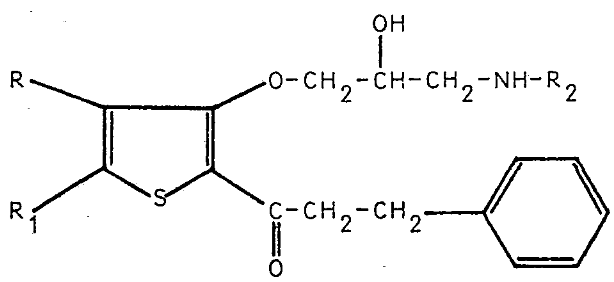 (I)

dans laquelle R et $R_1$ représentent chacun l'hydrogène ou un groupe méthyle et $R_2$ représente un groupe n-propyle, n-butyle, isobutyle ou tert-butyle, et leurs sels d'addition d'acides utilisables en thérapeutique.

2. Procédé pour la préparation des composés de formule générale I et de leurs sels d'addition d'acides utilisables en thérapeutique, caractérisé en ce que l'on fait réagir un composé de formule générale II

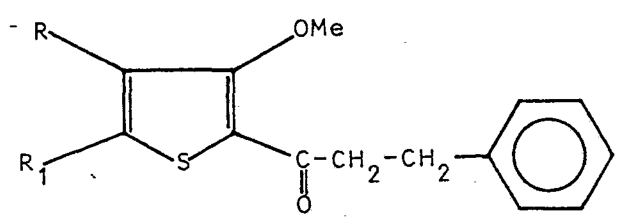 (II)

dans laquelle R et $R_1$ ont la signification ci-dessus et Me représente un cation de métal alcalin avec l'épichlorhydrine de formule III

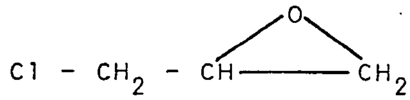

(III)

et on fait réagir le composé de formule générale IV ainsi obtenu

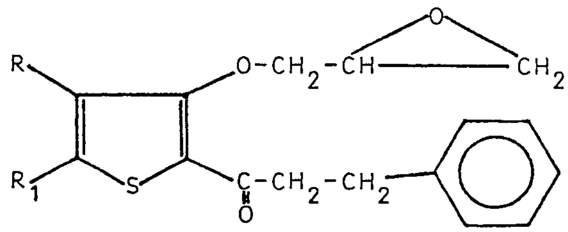

(IV)

dans laquelle R et $R_1$ ont la signification ci-dessus, avec une alkylamine de formule générale V

$$R_2—NH_2 \quad (V)$$

dans laquelle $R_2$ a la signification ci-dessus et on transforme éventuellement la base de formule générale I ainsi obtenue en un sel d'addition d'acide utilisable en thérapeutique.

3. Procédé selon la revendication 2, caractérisé en ce que l'on effectue la réaction du composé II avec l'épichlorhydrine III dans un solvant inerte vis-à-vis de la réaction.

4. Procédé selon la revendication 3, caractérisé en ce que l'on effectue la réaction dans l'épichlorhydrine en excès à une température comprise entre 110 et 140 °C.

5. Procédé selon la revendication 2, caractérisé en ce que l'on effectue la réaction entre le composé IV et l'alkylamine V dans un solvant inerte vis-à-vis de la réaction.

6. Procédé selon la revendication 5, caractérisé en ce que l'on effectue la réaction dans l'amine V en excès à une température comprise entre 50 et 100 °C.

7. Préparation pharmaceutique, caractérisée en ce qu'elle contient une proportion thérapeutiquement efficace d'un composé de formule générale I, en association avec un support ou diluant acceptable en pharmacie.